# EUROPEAN PATENT APPLICATION

(11) **EP 4 809 946 A1**
(43) Date of publication of application: **23.09.2026**
(21) Application number: 24891539.9
(22) Date of filing: 19.01.2024
(51) Int. Cl.: A61B 17/34, A61B 90/00, A61B 17/00

(54) **AUTOMATIC MULTICHANNEL HAIR TRANSPLANTER HAVING DETACHABLE MOTORIZED MODULE**

(30) Priority: 16.11.2023 KR 20230159374; 26.12.2023 KR 20230191309
(71) Applicant: OHDAE Corporation, Dalseong-gun, Daegu 42984 (KR)
(72) Inventor: KIM, Byoung Sul, Yeongyang-gun Gyeongsangbuk-do 36517 (KR); PARK, Eun Hun, Daegu 41492 (KR); PARK, Jeong Won, Daegu 42644 (KR)
(74) Representative: Grünecker Patent- und Rechtsanwälte PartG mbB
(86) International application number: PCT/KR2024/000968
(87) International publication number: WO 2025/105590

(57) **Abstract**

The present disclosure relates to a multi-channel hair transplanter provided with a plurality of needle channels, wherein the multi-channel hair transplanter includes: a needle channel bundle B including a bundle core member 1100 and a plurality of needle channels which are slidably coupled to a radial outside of the bundle core member; a hair transplanter main body A configured to accommodate the needle channel bundle B; and an electrification module C attachably and detachably coupled to the hair transplanter main body A.

## Description

### [Technical Field]

The present disclosure relates to a hair transplanter, and more particularly, to a multi-channel hair transplanter including a plurality of hair transplantation needles in the form of a bundle.

### [Background Art]

A hair transplanter is a medical device that is used in a hair surgical technique for transplanting hairs by harvesting hairs from a hair growth area of a scalp and implanting hairs into bald areas of the scalp.

Korean Patent Registration No. 10-2048492 (titled "Hair transplanter having a plurality of needle channels arranged in radial shape") disclosed, as a related-art transplanter, a multi-channel hair transplanter having a plurality of needle channels mounted therein, wherein each needle channel includes a needle (transplantation needle) and a core shaft slidably inserted into the needle.

However, such a related-art multi-channel hair transplanter is a manual device. Accordingly, a user (an operator such as a doctor) should grip a handle of the hair transplanter and should perform an operation of pushing down a main body of the transplanter every time the user implants hair follicles into a scalp one by one. Such an operation may physically burden the user when the user performs a typical hair transplantation operation to transplant thousands of hair follicles for several hours, and may decrease user's concentration, and may have great influence on success or failure in the operation.

In addition, in order to replace or cleanse inner consumables of a related-art hair transplanter, a user should separate an upper cap from a hair transplanter case and then should draw out inner components. However, a multi-channel hair transplanter may have a complicated inner structure and thus there may be inconvenience that it is not easy to dissemble or reassemble the hair transplanter.

### [Detailed Description of the Present disclosure]

### [Technical Objects]

The present disclosure has been developed in order to solve the problems of the related-art technology described above, and an object of the present disclosure is to provide an automatic hair transplanter which can automatically perform an ascending and descending operation of a needle channel in a multi-channel hair transplanter.

Another object of the present disclosure is to provide a hair transplanter which has an electrification module for automatically performing an ascending and descending operation of a needle channel easily attached to or detached from a main body thereof.

### [Technical Solving Means]

According to an embodiment of the present disclosure, there is provided a multi-channel hair transplanter provided with a plurality of needle channels, the multi-channel hair transplanter including: a needle channel bundle B including a bundle core member and a plurality of needle channels which are slidably coupled to a radial outside of the bundle core member; a hair transplanter main body A configured to accommodate the needle channel bundle B; and an electrification module C attachably and detachably coupled to the hair transplanter main body A.

In an embodiment, the hair transplanter main body A may include: a cylindrical case 10 configured to accommodate the needle channel bundle B; and an elevation gear holder interposed between the needle channel bundle B and the case and movable in a vertical direction relative to the case.

In an embodiment, the electrification module C may include: a driving unit; a first rotation member connected to a rotation shaft of the driving unit; a second rotation member installed below the first rotation member, spaced apart from the first rotation member; a power transmission member wound around the first and second rotation members to form a closed loop, and configured to transmit a rotational power of the first rotation member to the second rotation member; and a sliding member coupled to the power transmission member to be movable in the vertical direction by driving by the driving unit.

In an embodiment, the first rotation member and the second rotation member may be pullies, respectively, and the power transmission member may be a wire. In an alternative embodiment, the first rotation member and the second rotation member may be sprockets, respectively, and the power transmission member may be a chain.

In an embodiment, the electrification module C may include a driving unit; a screw shaft disposed in the vertical direction and rotated by driving by the driving unit; and a sliding member slidably coupled to the screw shaft and movable in the vertical direction by driving by the driving unit.

### [Advantageous Effects]

According to an embodiment of the present disclosure, there is provided an automatic multi-channel hair transplanter which is capable of automatically performing an ascending and descending operation of a needle channel.

According to an embodiment of the disclosure, since an electrification module is easily attached to or detached from a hair transplanter main body, assembly easiness of the hair transplanter can be enhanced.

### [Brief Description of the Drawings]

FIG. 1 is a perspective view of a multi-channel hair transplanter according to an embodiment of the disclosure;
FIG. 2 is an exploded perspective view of the multi-channel hair transplanter according to an embodiment;
FIG. 3 is a view provided to explain a needle channel bundle (B) according to an embodiment;
FIG. 4 is a perspective view and a cross-sectional view of a needle channel according to an embodiment;
FIG. 5 is an exploded perspective view of a hair transplanter main body A according to an embodiment;
FIG. 6 is a view provided to explain a case of the hair transplanter main body A according to an embodiment;
FIG. 7 is a view provided to explain a coupling relationship between the case and an elevation gear holder according to an embodiment;
FIG. 8 is an exploded perspective view of an electrification module C according to an embodiment;
FIG. 9 is a view provided to explain an inner structure of a housing according to an embodiment;
FIG. 10 is a view provided to explain an external structure of the housing according to an embodiment;
FIG. 11 is a view provided to explain an operation of a sliding member according to an embodiment;
FIG. 12 is a view provided to explain a configuration of the sliding member according to an embodiment;
FIG. 13 is a view provided to explain a driving button according to an embodiment;
FIG. 14 is a view provided to explain a housing attaching/detaching configuration according to an embodiment; and
FIG. 15 is a view provided to explain an operation of a sliding member according to an alternative embodiment.

### [Best Mode for Embodying the Invention]

Exemplary embodiments will now be described more fully with reference to the accompanying drawings to clarify aspects, other aspects, features and advantages of the present disclosure. The exemplary embodiments may, however, be embodied in many different forms and should not be construed as limited to the exemplary embodiments set forth herein. Rather, the exemplary embodiments are provided so that this disclosure will be thorough and complete, and will fully convey the scope of the application to those of ordinary skill in the art.

It will be understood that when an element is referred to as being "on" (or "under", "on the right of", or "on the left of") another element, the element can be directly on (or "under", "on the right of", or "on the left of") another element or a third element therebetween. In the drawings, lengths or thicknesses of elements are exaggerated for effective explanation of technical features.

Such expressions as "upper", "lower", "left", "right", "front", "rear", etc. used in the specification to explain a position relationship between elements do not mean directions or positions as an absolute criterion, and are relative expressions used for the convenience of explanation with reference to a corresponding drawing when the present disclosure is explained with reference to each drawing.

If the terms such as 'first' and 'second' are used to describe elements, these elements should not be limited by such terms. These terms are used for the purpose of distinguishing one element from another element only.

As used herein, the singular forms are intended to include the plural forms as well, unless the context clearly indicates otherwise. It will be further understood that the terms "comprise" and/or "comprising," when used in this specification, do not preclude the presence or addition of one or more other components.

Hereinafter, exemplary embodiments will be described in greater detail with reference to the accompanying drawings. The matters defined in the description are provided to assist in a comprehensive understanding of the exemplary embodiments. However, it is apparent that the exemplary embodiments can be carried out by those of ordinary skill in the art without those specifically defined matters. In the description of the exemplary embodiment, certain detailed explanations of related art are omitted when it is deemed that they may unnecessarily obscure the essence of the inventive concept.

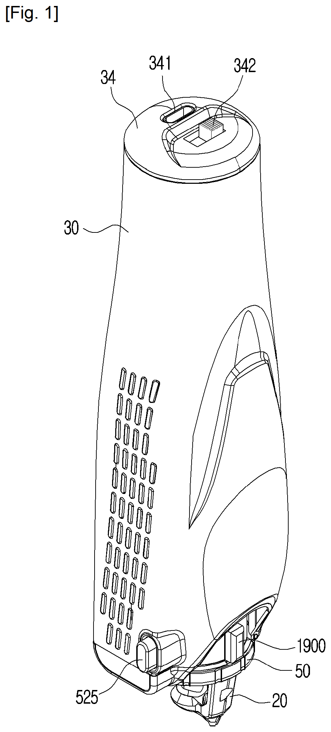

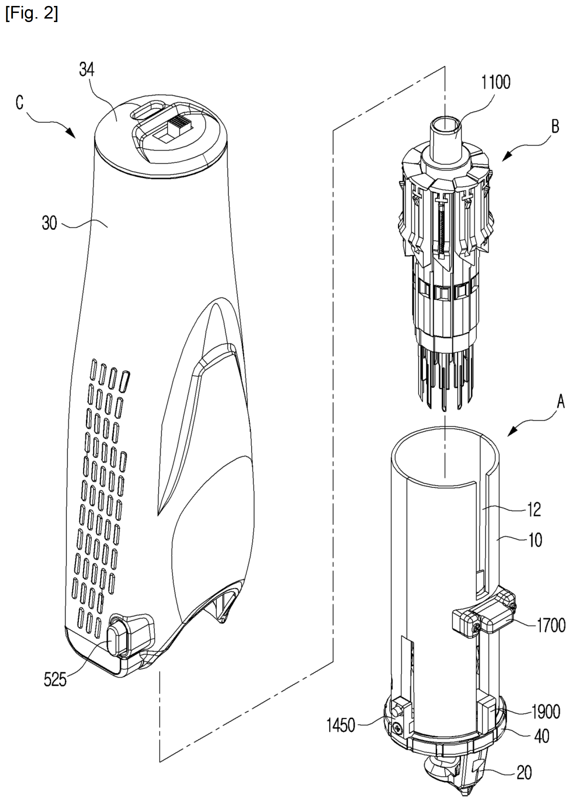

FIG. 1 is a perspective view of a multi-channel hair transplanter according to an embodiment of the disclosure, and FIG. 2 is an exploded perspective view of the multi-channel hair transplanter.

Referring to FIGS. 1 and 2, the multi-channel hair transplanter (hereinafter, referred to as a 'hair transplanter') according to an embodiment may include a hair transplanter main body A, a needle channel bundle B, and an electrification module C. In an embodiment, the hair transplanter may be assembled by attachably and detachably coupling the hair transplanter main body A, the needle channel bundle B, and the electrification module C.

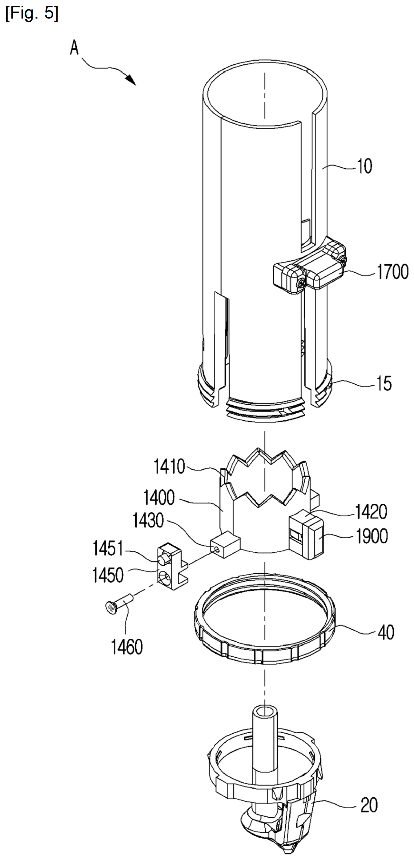

The hair transplanter main body A is a module that accommodates the needle channel bundle B, and may include a case 10, a nozzle portion 20 attached to a leading end of the case 10, and an elevation gear holder (1400 of FIG. 5) disposed in the case 10 for ascending or descending.

The case 10 is a cylindrical member that accommodates the needle channel bundle B. The elevation gear holder 1400 disposed in the case 10 is a cylindrical member, and may be disposed between the case 10 and the needle channel bundle B accommodated in the case 10 and may ascend and descend in a vertical direction relative to the case 10.

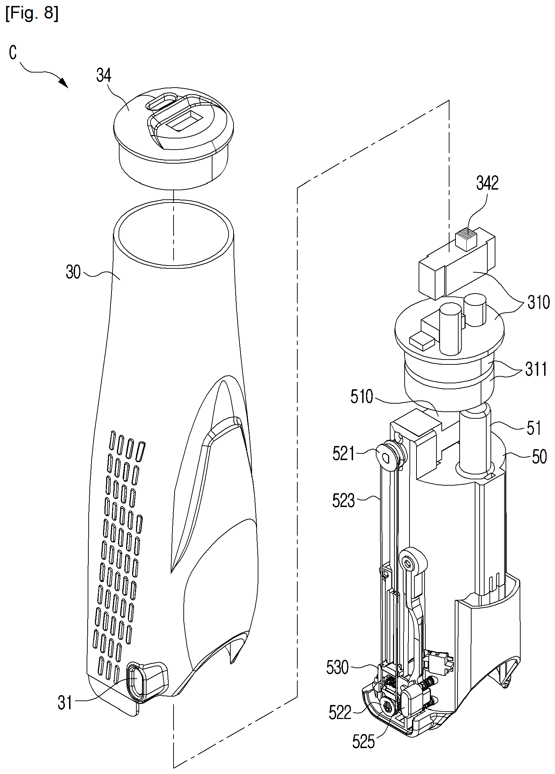

A connection member 1450 may be attached to at least one side surface of the elevation gear holder 1400. The connection member 1450 may be configured to protrude to the outside of the case 10, and may be attachably and detachably connected to a sliding member (530 of FIG. 8) of the electrification module C. Accordingly, as will be described below, according to a vertical movement of the sliding member 530 caused by a driving unit (510 of FIG. 8), the elevation gear holder 1400 coupled to the sliding member 530 may also move in the vertical direction. A detailed configuration of the hair transplanter main body A described above will be described hereinbelow with reference to FIGS. 5 to 7.

In the disclosure, each element's, such as the elevation gear holder 1400, the sliding member 530, a needle channel 1200, a needle 1220, a core shaft 1230, "ascending", 'retreating", or "moving back" refers to the corresponding element's moving in a direction away from a scalp, and each element's "descending" or "moving forward" refers to the corresponding element's moving toward a scalp.

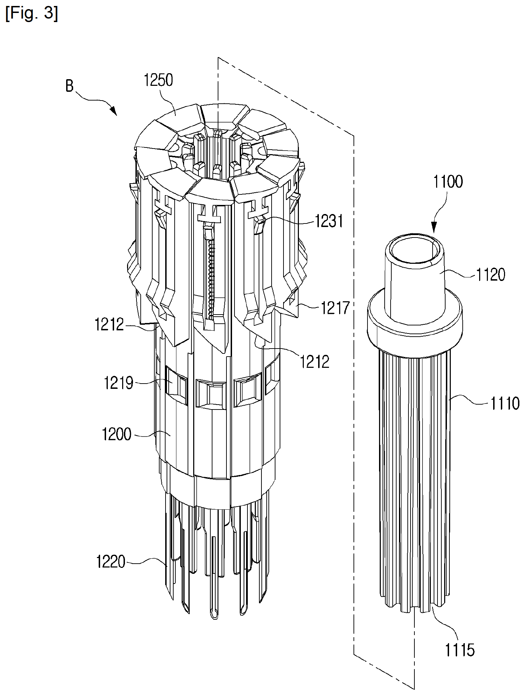

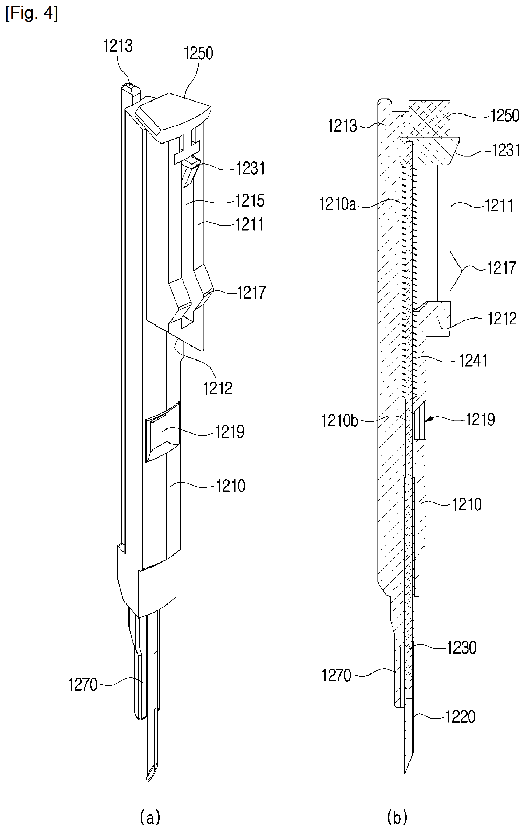

The needle channel bundle B may include a bundle core member 1100 and a plurality of needle channels 1200 coupled to an outer circumference of the bundle core member 1100 to be slidable in the vertical direction. Each of the needle channels 1200 may be slidably coupled to a radial outside of the bundle core member 1100. A needle 1220 may protrude from a leading end of each needle channel 1200. A core shaft (1230 of FIG. 4) may be embedded in the needle 1220 to be slidable in the vertical direction, and a hair follicle may be threaded through a leading end of the core shaft 1230, that is, a lower end of the core shaft 1230 in an inner space of the needle 1220, and the core shaft 120 may relatively descend with the needle 1220 being inserted into patient's scalp, and may perform hair transplantation by pushing a hair follicle into the scalp. A detailed configuration of the needle channel bundle B will be described with reference to FIGS. 3 and 4.

The electrification module C is a member that descends from the upper portion of the hair transplanter main body A and is attachably and detachably coupled to the hair transplanter main body A, and may have a driving unit to automatically raise or lower the elevation gear holder 1400. The electrification module C may include a cylindrical housing 30 and an upper cap 34 attached to an upper portion of the housing. The upper cap 34 may be provided with a terminal 341 (for example, a USB terminal) to communicate with an outside or to receive power from an outside, and a button 342 to power on or off the electrification module C.

In an embodiment, the electrification module C may include a driving button 525 to start an ascending and descending operation of the elevation gear holder 1400. For example, as shown in FIG. 1, the driving button 525 may be positioned on a position of user's thumb or index finger when the user (for example, a doctor) performs hair implantation by holding the hair transplanter, so that it is easy to manipulate the hair transplanter.

The driving button 525 may communicate with a controller (310 of FIG. 8) in the electrification module C wiredly or wirelessly, and, when a user presses the driving button 525, the sliding member 530 and the elevation gear holder 1400 coupled thereto may perform an ascending and descending operation under control of the controller 310.

In an alternative embodiment, instead of the driving button 525, a motion sensor or a touch sensor may be installed. When a user touches the motion sensor or the touch sensor with user's finger, the sensor may detect the finger and the controller 310 may perform an ascending and descending operation of the elevation gear holder 1400. In another alternative embodiment, the hair transplanter may include a gyro sensor instead of the driving button 525. The gyro sensor may detect a motion or a state of the hair transplanter (for example, at which angle the hair transplanter is erected or lies) in real time, and the controller 310 may perform an ascending and descending operation of the elevation gear holder 1400, based on a real-time detection signal of the gyro sensor.

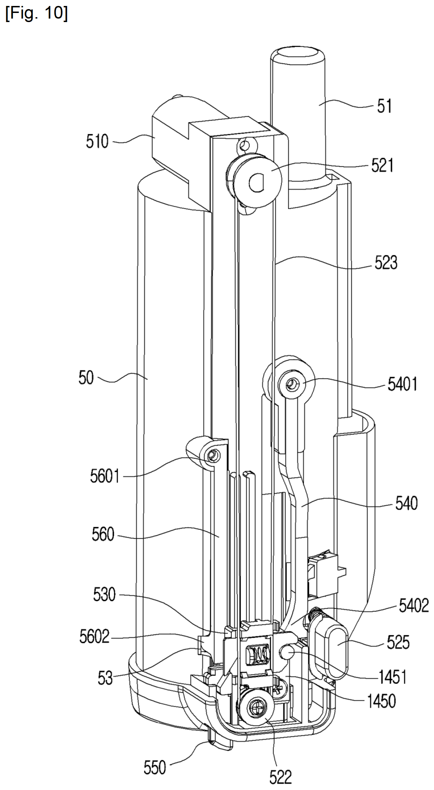

In an embodiment, the electrification module C may further include a disassembly button (550 of FIG. 10), and a user may dissemble the hair transplanter by pressing the disassembly button 550 as shown in FIG. 2. That is, as shown in FIG. 2, the electrification module C may be separated from the main body A of the hair transplanter, and the needle channel bundle B may be separated from the hair transplanter main body A by pulling up the bundle core member 1100 exposed from the upper portion of the hair transplanter main body A. Accordingly, a user can simply and swiftly replace the needle channel bundle B.

Hereinafter, the needle channel bundle B will be described with reference to FIGS. 3 and 4. FIG. 3 is a perspective view provided to explain the needle channel bundle B according to an embodiment, and FIG. 4 schematically illustrates a perspective view and a cross-sectional view of the needle channel bundle 1200.

Referring to FIG. 3, the needle channel bundle B may include the bundle core member 1100 and the plurality of needle channels 1200 coupled to the bundle core member 1100 to be slidable in the vertical direction.

In an embodiment, the bundle core member 1100 may include a cylindrical channel coupling portion 1110 and a head portion 1120 formed on an upper portion of the channel coupling portion 1110. The channel coupling portion 1110 and the head portion 1120 may be integrally formed with each other, or may be separately fabricated and coupled to each other. A penetrating hole may be formed in the channel coupling portion 1110 and the head portion 1120 in the vertical direction.

A plurality of slide rails 1115 may be formed on an outer circumference of the channel coupling portion 1110 in a radial direction from a center axis of the channel coupling portion 1110 at equal intervals. In an embodiment, the slide rail 1115 may be formed by engraving lengthwise in the vertical direction, and each needle channel 1200 may be inserted into and coupled with each slide rail 1115 to be slidable in the vertical direction.

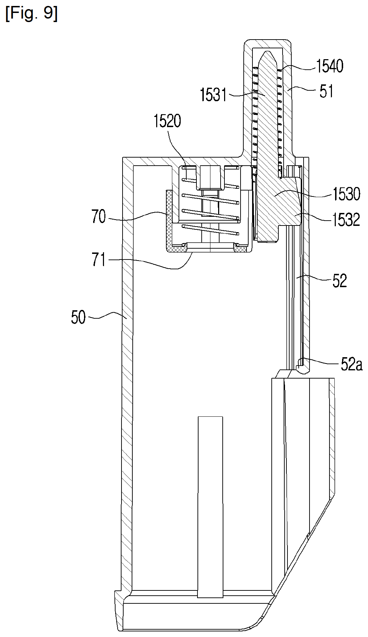

The head portion 1120 serves as a stopper to prevent the needle channels 1200 inserted into the channel coupling portion 1110 from further ascending. A diameter of the upper side of the head portion 1120 may be the same as or smaller than an inner diameter of a center penetrating hole 71 of a spring cover (70 of FIG. 9) of the electrification module C. Accordingly, when the electrification module C is coupled to the hair transplanter main body A, the head portion 1120 may be inserted into the penetrating hole 71 of the spring cover 70, and the spring cover 70 may elastically press down and support the bundle core member 1100.

Referring to FIGS. 3 and 4, the needle channel 1200 may include a body portion 1210 having a tubular inner space formed therein in the vertical direction, the needle 1220 for hair transplantation which is coupled to a lower portion of the body portion 1210, and the core shaft 1230 slidably disposed in inner spaces of the body portion 1210 and the needle 1220.

The tubular inner space of the body portion 1210 may include a first diameter portion 1210a formed on an upper portion thereof and having a relatively large diameter, and a second diameter portion 1210b formed on a lower portion thereof and having a relatively small diameter. The second diameter portion 1210b on the lower portion may have a diameter sufficient to allow the core shaft 1230 to pass therethrough, and the first diameter portion 1210a on the upper portion may have a diameter sufficient to accommodate a spring 1241 fitted over a circumference of the core shaft 1230. The needle 1220 may be inserted into and coupled to the body portion 1210 through an opened lower end of the second diameter portion 1210b of the body portion 1210. The needle 1220 has a cylindrical shape having a hollow formed in a center thereof, that is, a penetrating hole formed therein in a longitudinal direction, and the penetrating hole of the needle 1220 may fluidly communicate with the inner space of the body portion 1210.

The body portion 1210 may include a side protrusion 1213 formed along one side surface of the body portion lengthwise in the vertical direction. The side protrusion 1213 may protrude toward the channel coupling portion 1110, that is, in a radially inward direction, and a protruding shape of the side protrusion 1213 may be configured to be engaged with a recess shape of the slide rail 1115 of the channel coupling portion 1110. Accordingly, the side protrusion 1213 of the body portion 1210 is inserted into and engaged with the slide rail 1115 of the channel coupling portion 1110, such that the needle channel 1220 slides along the slide rail 1115 in the vertical direction.

The needle channel 1200 may include a locking protrusion 1211 extended from the body portion 1210 in the opposite direction of the side protrusion 1213, that is, in a radially outward direction of the needle channel bundle B. The locking protrusion 1211 may include a slot 1215 formed in the vertical direction, a projection 1217 extended from a surface of the locking protrusion 1211 to further protrude in the radially outward direction, and toothed ratchet gear teeth formed on a lower surface of the locking protrusion 1211 and having an inclination angle in one direction.

The slot 1215 may be formed on the body portion 1210 in the longitudinal direction (vertical direction) and may be opened toward one side surface of the body portion 1210. The slot 1215 may be formed on a surface of the locking protrusion 1211 in the radially outward direction in the vertical direction, and may fluidly communicate with the tubular inner space (that is, a space of the first diameter portion 1210a) of the body portion 1210.

The core shaft 1230 may be disposed in the inner space of the body portion 1210, and a lever 1231 may be coupled to an upper end of the core shaft 1230. The lever 1231 may be guided by the slot 1215 and may slide in the vertical direction. The spring 1241 may be fitted over the circumference of the core shaft 1230. An upper end of the spring 1241 may be supported on the lever 1231, and a lower end of the spring 1241 may be supported on a stepped portion between the first diameter portion 1210a and the second diameter portion 1210b in the body portion 1210.

The slot 1215 may have an opened upper end, and an upper cap 1250 may be inserted into and coupled with the opened upper end. The upper cap 1250 may serve as a stopper to prevent the lever 1231 from further moving up. As shown in FIG. 3, an upper surface of the upper cap 1250 may have a surface that is inclined in one direction to allow the needle channel bundle B to smoothly rotate. That is, the upper surface of the upper cap 1250 may be inclined downwardly in a rotation direction of the needle channel bundle, such that a push bar (1530 of FIG. 9) contacting the upper surface of the upper cap 1250 does not interfere with rotation of the needle channel bundle B.

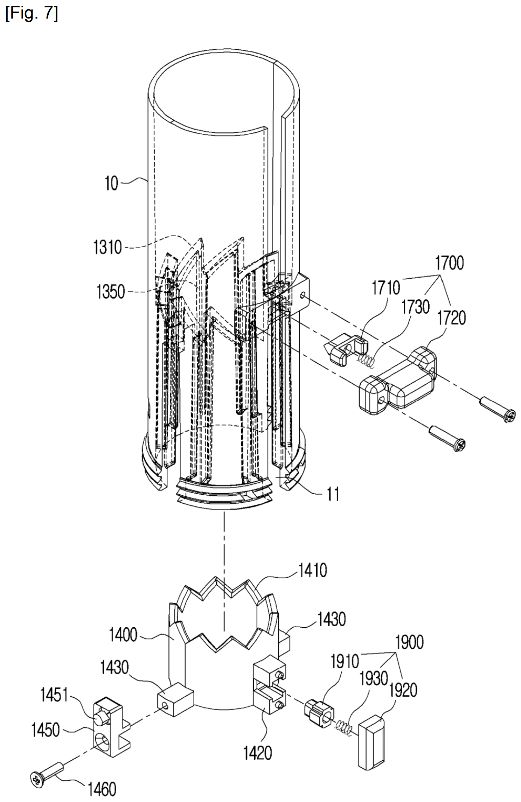

The body portion 1210 of the needle channel 1200 may further include a locking recess 1219. The locking recess 1219 may be formed on a surface of the body portion 1210 that faces in the radially outward direction, under the locking protrusion 1211. The locking recess 1219 may partially accommodate a latch (1910 of FIG. 7) of a needle channel locking portion 1900.

In an embodiment, the body portion 1210 may further include a rotor guide 1270 extended downwardly therefrom. The rotor guide 1270 may be extended downwardly from a lower end of the body portion 1210 and may be configured to partially enclose an outer circumference of the needle 1220. For example, in the embodiment illustrated in the drawings, the rotor guide 1270 may be configured to enclose an approximately half circumference (for example, 180 degrees) of the outer circumference (that is, 360 degrees) of the needle 1220. However, this is merely an example. In an alternative embodiment, the rotor guide 1270 may be configured to enclose the needle 1220 between 90 degrees and 270 degrees of the entire circumference (360 degrees) of the needle 1220.

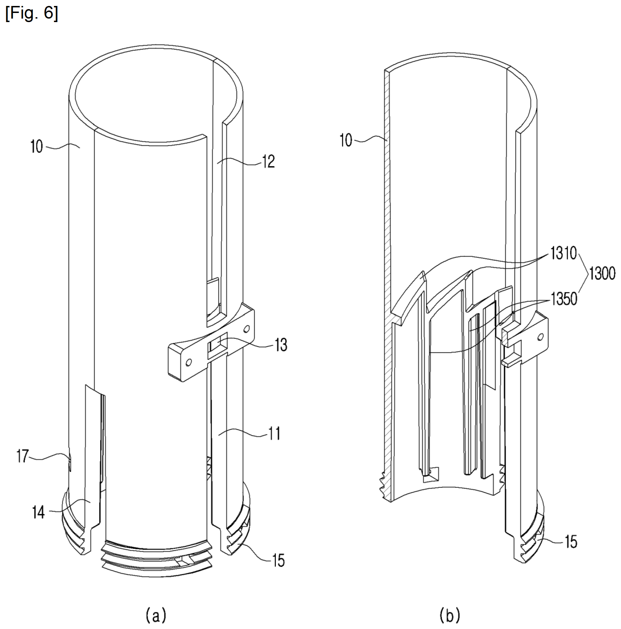

Hereinafter, an exemplary configuration of the hair transplanter main body A will be described with reference to FIGS. 5 to 7. FIG. 5 is an exploded perspective view of the hair transplanter main body A according to an embodiment, FIG. 6 is a view provided to explain the case of the hair transplanter main body A, and FIG. 7 is a view provided to explain a coupling relationship between the case and the elevation gear holder according to an embodiment.

Referring to FIG. 5, the hair transplanter main body A according to an embodiment may include the cylindrical case 10, the nozzle portion 20, a securing ring 40, and the elevation gear holder 1400.

The case 10 is a cylindrical member that has an upper end and a lower end opened and has a hollow formed therein to accommodate the needle channel bundle B and the elevation gear holder 1400. A core shaft locking portion 1700 may be attached to one side surface of the case 10. The core shaft locking portion 1700 may serve to catch the core shaft 1230 slidably disposed in the needle channel 1200, and to temporarily prevent the core shaft 1230 from being moved.

Referring to FIGS. 5 and 6, the core shaft locking portion 1700 may be attached approximately to a middle height of the case 10, and a first slot 11 and a second slot 12 may be formed on a side surface of the case 10 on a lower side and an upper side of a position where the core shaft locking portion 1700 is installed, wherein the first slot 11 and the second slot 12 are formed in the longitudinal direction. The first slot 11 may be formed to prevent a first connection protrusion piece 1420 protruding from the elevation gear holder 1400 in the radially outward direction from interfering with the case 10 when the first connection protrusion piece 1420 moves in the vertical direction, and the second slot 12 may guide the push bar (1530 of FIG. 9) to be inserted and moved in the vertical direction, and simultaneously, may prevent the push bar 1530 from interfering with the case 10.

The nozzle portion 20 may be secured to a lower end of the case 10. For example, the nozzle portion 120 may be attached to the case 10 by means of the securing ring 40. In the illustrated embodiment, inner screw threads of the securing ring 40 and outer screw threads 15 of the lower end of the case 10 may be configured to be engaged with each other, and an annular frame of the nozzle portion 20 may be inserted into the inside of the lower end of the case 10, and in this state, the securing ring 40 may be secured to the lower end of the case 10, and, by pressing the nozzle portion 20, the nozzle portion 20 may be secured to the case 10.

A nozzle hole of the nozzle portion 20 may be a penetrating hole through which the needle 1220 of one needle channel 1200 passes, and specifically, the nozzle hole may be formed to allow the needle 1220 of the needle channel and the rotor guide 1270 formed around the needle to pass therethrough.

The nozzle hole of the nozzle portion 20 may be aligned with the push bar 1530 along one straight line. Accordingly, when the push bar 1530 pushes down one needle channel 1200, the needle 1220 of the pushed needle channel 1220 protrudes downward from the hair transplanter through the nozzle hole by a predetermined length.

The elevation gear holder 1400 may be a member that is interposed between the needle channel bundle B and the inner surface of the case 10, and is configured to ascend and descend in the vertical direction relative to the case 10. The elevation gear holder 1400 may have a cylindrical shape to enclose the circumference of the needle channel bundle B, and may have a wedge protrusion 1410 formed an edge of an upper end thereof.

The elevation gear holder 1400 may include the first connection protrusion piece 1420 and a second connection protrusion piece 1430 which protrude from the outer circumference thereof in the radial direction. The first connection protrusion piece 1420 may protrude to allow the needle channel locking portion 1900 to be attached thereto, and the second connection protrusion piece 1430 may be formed to allow the connection member 1450 to be attached thereto. The elevation gear holder 1400 may be integrally connected with the sliding member (530 of FIG. 8) of the electrification module C through the connection member 1450. In addition, the first slot 11 and a third slot 14 may be formed on a side surface of the case 10 to prevent the case 10 from interfering with vertical movements of the first connection protrusion piece 1420 and the second connection protrusion piece 1430.

The elevation gear holder 1400 is a substantially cylindrical member, and may be disposed to enclose the outer circumference of the needle channel bundle B. The surface of the upper end of the cylindrical elevation gear holder 1400 may be formed to contact a lower end surface of the locking protrusion 1211 of the needle channel 1200, that is, a lower surface of the ratchet gear teeth 1212. That is, the wedge protrusions 1410 having a sharpened shape may be continuously formed on the upper surface of the elevation gear holder 1400, and the same number of wedge protrusions 1410 as the number of the ratchet gear teeth 1212 of the needle channel bundle B may be formed to be engaged with the plurality of ratchet gear teeth 1212 of the needle channel bundle B.

Referring to FIGS. 6B and 7, a ratchet support 1300 may be formed on an inner surface of the case 10 by carving. The ratchet support 1300 may protrude inward from an inner surface of the case 10.

The ratchet support 1300 may protrude from the inner surface of the case 10 to enclose the inner surface of the case 10 once. The ratchet support 1300 may not be formed on positions of the first slot 11 and the third slot 14 formed on predetermined side surface areas of the case 10 at least in part in order to prevent interference with the first and second connection protrusion pieces 1420, 1430 of the elevation gear holder 1400.

The ratchet support 1300 may have an upper surface 1310 which protrudes in the form of a wedge by carving, and an elevation guide 1350 which protrudes in the vertical direction by carving. The upper surface 1310 in the wedge shape may be configured to contact a lower surface of the ratchet gear teeth 121 of the needle channel 1200. That is, the upper surface 1310 of the ratchet support 1300 may be formed in a toothed shape having an inclination angle in one direction to be engaged with the ratchet gear teeth 1212. The elevation guide 1350 may guide the elevation gear holder 1400 to perform an ascending and descending operation without being shaken in a gap space in the case 10.

In an embodiment, the core shaft locking portion 1700 may be attached to one side surface of the case 10. The core shaft locking portion 1700 may be disposed on a radially outward surface of the needle channel 1200 that is pushed down by the push bar 1530. The shaft core locking portion 1700 may serve to temporarily stop the core shaft 1230 in the needle channel 1200 not to ascend while the needle channel 1200 is ascending after being pushed down by the push bar 1530.

Referring to FIG. 7, the core shaft locking portion 1700 may include a latch 1710, a latch receiver 1720 to receive the latch 1710, and an elastic member 1730 interposed between the latch 1710 and the latch receiver 1720. The latch receiver 1720 may be fixed to a side surface of the case 10 by means of bolts through bolt recesses of extension areas of left and right sides thereof. The latch receiver 1720 may have a space into which the latch 1710 is inserted, and the elastic member 1730 such as a spring may be interposed in this space. The latch 1710 may pass through a penetrating hole 13 of the case 10 to lock the lever 1231 of the needle channel 1200 in the case 10.

When no force is applied to the latch 1710, the latch 1710 may be ejected from the latch receiver 1720 (in a radially inward direction of the needle channel bundle B), and may come into contact with the lever 1231 when the lever 1231 slides in the vertical direction. When the lever 1231 descends above the latch 1710, the lever 1231 may descend while pushing the latch 1710 toward the latch receiver 1720, but, when the lever 1231 ascends below the latch 1710, an upper surface of the lever 1231 may be blocked by the latch 1710 and may not ascend.

The projection 1217 may be formed on a side surface of the slot 1215 of the needle channel 1200, and may protrude in the radially outward direction of the needle channel bundle B. The projection 1217 may protrude in the radially outward direction to the same degree as the lever 1231 or more outwardly than the lever 1231. A horizontal width of the latch 1710 may be set to interfere not only with the lever 1231 but also with the vertical movement of the projection 1217.

Accordingly, whatever the needle channel 1200 ascends below the latch 1710 or descends above the latch 1710, the needle channel 1200 may ascend and descend while the projection 1217 is pushing the latch 1710 toward the latch receiver 1720. Accordingly, when the body portion 1210 of the needle channel 1200 ascends in a state in which the upper surface of the lever 1231 is caught by the latch 1710 and the lever 1231 does not ascend, the projection 1217 may ascend while pushing the latch 1710 toward the latch receiver 1720, and in this case, the lever 1231 may be unlocked and the core shaft 1230 may ascend along with the body portion 1210.

The needle channel locking portion 1900 may be a device that catches one needle channel 1200 which descends by being pushed by the push bar 1530, and causes the needle channel 1200 to move according to a vertical movement of the elevation gear holder 1400.

In an embodiment, the needle channel locking portion 1900 may include a latch 1910, a latch receiver 1920 to receive the latch 1910, and an elastic member 1930 interposed between the latch 1910 and the latch receiver 1920. The latch receiver 1920 may be coupled to the first connection protrusion piece 1420 of the elevation gear holder 1400 by press-fitting or coupling such as bolt screwing. The latch receiver 1920 may have a space into which the latch 1910 is inserted, and the elastic member 1930 such as a spring may be interposed in this space. The latch 1910 may elastically move in the radially inward direction, and may be engaged with the locking recess 1219 of the needle channel 1200, thereby temporarily preventing the needle channel 1200 from ascending.

Due to this configuration, the needle channel 1200 may also move according to the vertical movement of the elevation gear holder 1400 in a locked state in which the latch 1910 is inserted into the locking recess 1219. However, in this locked state, when the needle channel bundle B rotates in the counter clockwise direction, the latch 1910 may be retreated toward the latch receiver 1920 and may be unlocked, and, when the needle channel bundle B rotates as much as one channel, the latch 1910 may be locked into the locking recess 1219 of another needle channel 1200.

Due to the above-described configuration, the needle channel bundle B may rotate by a predetermined angle as much as one channel every time the elevation gear holder 1400 performs an ascending and descending operation one time. The rotation of the needle channel bundle B and the ascending and descending operation of the needle channel 1200, and the ascending and descending operation of the core shaft 1230, which are performed by the ratchet support 1300, the elevation gear holder 1400, the core shaft locking portion 1700, and the needle channel locking portion 1900, are disclosed in Korean Patent Registration No. 10-2048492 (titled "Hair transplanter having a plurality of needle channels arranged in radial shape"), and thus a detailed description thereof is omitted.

FIG. 8 is an exploded perspective view of the electrification module C according to an embodiment. Referring to FIG. 8, the electrification module C may be attachably and detachably coupled to an upper portion of the hair transplanter main body A, and may have a driving unit to raise and lower the elevation gear holder 1400 of the hair transplanter main body A. In an embodiment, the electrification module C may include an external housing 30, an internal housing 50, and an upper cap 34.

The external housing 30 may be a cylindrical member that encloses the internal housing 50, and the upper cap 34 may be attached to an upper end of the external housing. The internal housing 50 is a cylindrical member that has an upper portion closed, and may have an inner space to accommodate the hair transplanter main body C. A driving unit 510, a controller 310, and a battery 311 may be disposed on the closed upper portion of the internal housing 50.

The controller 310 is to control operations of the driving unit 510, and for example, may be implemented by a PCB or a micro-processor mounted thereon. The battery 311 may be, for example, a battery of a coin cell type, and may supply power to the controller 310 and the driving unit 510. In an alternative embodiment, the battery 311 may be omitted and power may be supplied from an outside.

A space may be formed between the internal housing 50 and the external housing 30 to have some components of the electrification module C disposed therein. For example, in the illustrated embodiment, components such as the sliding member 530 and a power transmission member to raise and lower the sliding member may be disposed in the space, and such components may be attached to an outer surface of the internal housing 50 or may be attached to an inner surface of the external housing 30.

In an embodiment, the driving unit 510 may be an electric motor, but this should not be considered as limiting, and the driving unit 510 may be implemented by other driving sources driven by electricity. In the illustrated embodiment, a first rotation member may be directly or indirectly (via a gear box) installed on a rotation shaft of the driving unit 510, and a second rotation member may be installed below the first rotation member, spaced apart therefrom.

A power transmission member may further be installed between the first rotation member and the second rotation member to transmit rotational power of the first rotation member to the second rotation member. The power transmission member may be wound around the first and second rotation members, forming a closed loop and transmitting rotational power of the first rotation member to the second rotation member. In addition, the sliding member 530 may be coupled to the power transmission member, and may be moved in the vertical direction by driving by the driving unit 510.

In the illustrated embodiment, the first rotation member may be implemented by a first pulley 521, the second rotation member may be implemented by a second pulley 522, and the power transmission member may be implemented by a wire 523. However, the first and second rotation members and the power transmission member are not limited to the above-described components, and for example, in another alternative embodiment, the first rotation member and the second rotation member may be sprockets, respectively, and the power transmission member may be a chain.

FIG. 9 is a cross-sectional view schematically illustrating an inner structure of the internal housing 50. Referring to FIG. 9, the internal housing 50 may be a cylindrical member that has a lower portion opened, and may include a spring 1520 disposed in an upper portion of an inside thereof, the spring cover 70, and the push bar 1530.

The push bar 1530 may be guided by a guide portion 52 formed on an inner surface of the internal housing 50 in the longitudinal direction, and may move in the vertical direction. The push bar 1530 may serve to push down one needle channel 1200 of the needle channel bundle B in a state in which the electrification module C is coupled to the hair transplanter main body A.

The push bar 1530 may include a spring insertion portion 1531 extended upward therefrom, and the spring 1540 may be fitted over the spring insertion portion 1531. A spring receiver 51 which is an inner space further extended upward may be formed above the upper portion of the push bar 1530 to partially receive the spring 1540 and the spring insertion portion 1531 inserted into the spring. That is, when the push bar 1530 ascends to the maximum, the spring insertion portion 1531 of the push bar 1530 may ascend to the inside of the spring receiver 51 (that is, the state illustrated in FIG. 9), and, when the push bar 1530 descends due to elasticity of the spring 1540, the push bar 1530 may descend to a locking projection 52a of a lower end of the guide portion 52. The locking projection 52a may protrude inward the internal housing 50, and may serve as a stopper against a downward movement of the push bar 1530, and may also prevent the push bar 1530 from being released from the guide portion 52.

The spring 1520 may be disposed in a center of the upper portion of the inside of the internal housing 50. The spring cover 70 may be elastically attached to the internal housing 50 while covering a lower portion of the spring 1520. The penetrating hole 71 may be formed on a center of the spring cover 70, and may have a diameter sufficient to allow the bundle core member 1100 of the needle channel bundle B to be inserted thereinto. Accordingly, when the electrification module C is coupled to the hair transplanter main body A, the upper end of the bundle core member 1100 of the needle channel bundle B may be inserted into the penetrating hole 71 of the spring cover 70, and in this state, the spring cover 70 may serve to press down the needle channel bundle B elastically by means of the spring 1520, thereby allowing the needle channel bundle B to be securely positioned in the hair transplanter main body A without shaking.

In a preferred embodiment, when the needle channel bundle B is replaced or sterilized, the electrification module C may be detached from the hair transplanter main body A as shown in FIG. 2, such that the hair transplanter may be disassembled by modules. That is, when a user pushes the disassembly button (550 of FIG. 10) installed at a lower end of the electrification module C with user's hand, the electrification module C and the hair transplanter main body A may be decoupled from each other, and the electrification module C may be separated from the hair transplanter main body A, and then, the needle channel bundle B accommodated in the hair transplanter main body A may be drawn out.

In this case, the push bar 1530 may be inserted into the guide portion 52 without being released downward due to the locking projection 52a of the guide portion 32, and the spring cover 70 may be elastically attached to the internal housing 50. Accordingly, when the electrification module C is separated from the hair transplanter main body A as shown in FIG. 2, components of the electrification module C such as the push bar 1530 or the spring 1520 may not be released from the electrification module C.

Hereinafter, installation and operation structures of the sliding member 530, and a configuration for attaching or detaching the electrification module C to or from the hair transplanter main body A will be described with reference to FIGS. 10 to 14. In embodiments described below, it is assumed that the first and second rotation members are pullies 521, 522 and the power transmission member is a wire 523.

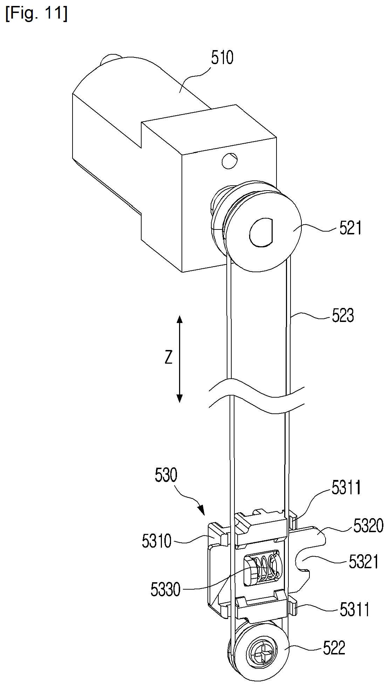

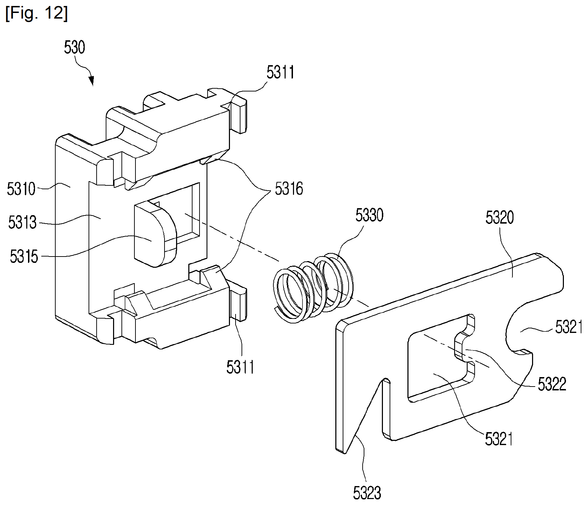

FIG. 10 illustrates the electrification module C when the external housing 30 is removed, that is, exterior components of the internal housing 50 of the electrification module C. For the convenience of explanation, components such as the controller 310, the battery 311 are not illustrated. FIG. 11 schematically illustrates only components needed to drive the sliding member 530, and FIG. 12 is a perspective view of the sliding member 530.

Referring to FIGS. 10 and 11, the driving unit 510 may be disposed on an upper portion of the internal housing 50, and the first pully 521 may be directly or indirectly connected to a driving shaft of the driving unit. The second pully 522 may be disposed on a lower portion of the internal housing 50, and the wire 523 may be wound between the first and second pullies 521, 522 as a power transmission member. The sliding member 530 may be coupled to a line of one side of the wire 523, and accordingly, the sliding member 530 may move in the vertical direction as the wire 523 is moved by the driving unit 510.

Referring to FIG. 12, the sliding member 530 may include a main body 5310, a locking plate 5320, and a spring 5330 interposed between the main body and the locking plate. The main body 5310 may include a wire fixing portion 5311 coupled with the wire 523, and, for example, the main body 5310 may be fixed to the wire 523 by winding the wire 523 around the wire fixing portion 5311 once or more.

A locking plate seating portion 5313 may be formed on a front surface of the main body 5311 to have the locking plate 5320 placed thereon. The locking plate 5320 is a plate-shaped member that has a penetrating hole 5325 formed on a center thereof, and may be inserted between the locking plate seating portion 5313 and a guide piece 5316, and may be coupled to the main body 5310 to be slidable horizontally. The locking plate seating portion 5313 may include a spring support portion 5315 protruding from a surface thereof toward the penetrating hole 5325 of the locking plate 5320, and one end of the spring may be supported on the spring support portion 5315 when the spring 5330 is inserted into the penetrating hole 5325. In this case, the other end of the spring 5330 may be fitted over a spring insertion portion 5322 protruding inward the penetrating hole 5325 of the locking plate 5320, such that the spring 5330 is prevented from being released.

A locking recess 5321 may be formed on one side surface of the locking plate 5320. As shown in FIG. 10, a locking projection 1451 of the connection member 1450 coupled to the elevation gear holder 1400 may be inserted into the locking recess 5321. Accordingly, in this state in which the locking projection 1451 is locked into the locking recess 5321, when the sliding member 530 moves in the vertical direction, the elevation gear holder 1400 may also move in the vertical direction.

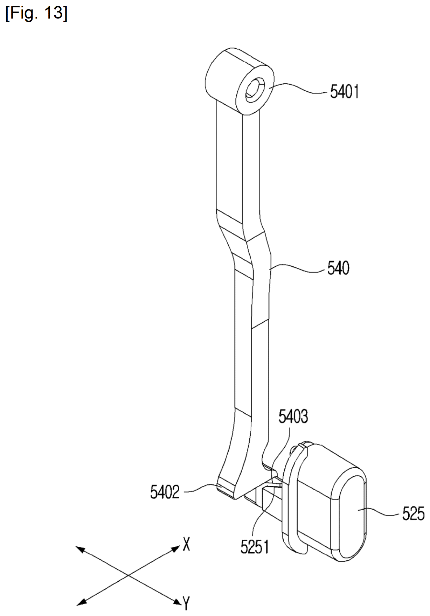

FIG. 13 schematically illustrates the driving button 525 installed on an outside of the internal housing 50, and a stopper member 540. Referring to FIGS. 10 and 13, the internal housing 50 may include the stopper member 540 which is disposed adjacent to a moving path along which the sliding member 530 moves in the vertical direction, and the driving button 525 which is disposed in the proximity of the lower end of the stopper member 540. In an embodiment, the stopper member 540 may have an elongated bar shape. An upper end 5401 of the stopper member 540 may be coupled to the internal housing 50. A protrusion 5402 may protrude from a lower end of the stopper member 540 toward the sliding member 530. Accordingly, as shown in FIG. 10, in a state in which the sliding member 530 descends to the lowest end, the protrusion 5402 may be disposed to interfere with an ascending path of the sliding member 530. For example, in the embodiment illustrated in FIG. 10, the protrusion 5402 may be positioned on an upper portion of the locking plate 5320 of the sliding member 530 to prevent the sliding member 530 from ascending.

As shown in FIG. 13, an inclined surface 5403 may be formed on a lowest end of the stopper member 540, and an inclined surface 5251 may be formed on the driving button 525 to face the inclined surface 5403. Accordingly, when a user presses the driving button 525, the driving button 525 moves along the Y axis of FIG. 13 in a direction close to the stopper member 540, and the bar-shaped stopper member 540 structurally has elasticity, and accordingly, the protrusion 5402 of the stopper member 540 moves along the X axis in a direction away from the sliding member 530.

As described above, when the user presses the driving button 525, the protrusion 5402 may be released from the upper portion of the sliding member 530, such that the sliding member 530 moves up without interference by the protrusion 5402. In the illustrated embodiment, the inclined surfaces 5403, 5251 facing each other implement this operation, but this is merely an example. Other methods may be implemented to move the protrusion 5402.

The driving button 525 and the controller 310 may be wiredly or wirelessly connected to electrically communicate with each other although this is not illustrated. When the driving button 525 is pressed, an electrical signal may be transmitted to the controller 310. Accordingly, when a user presses the driving button 525, the protrusion 5402 may be retreated from the sliding member 530, and simultaneously, the driving unit 510 may be driven under control of the controller 310, thereby raising the sliding member 530.

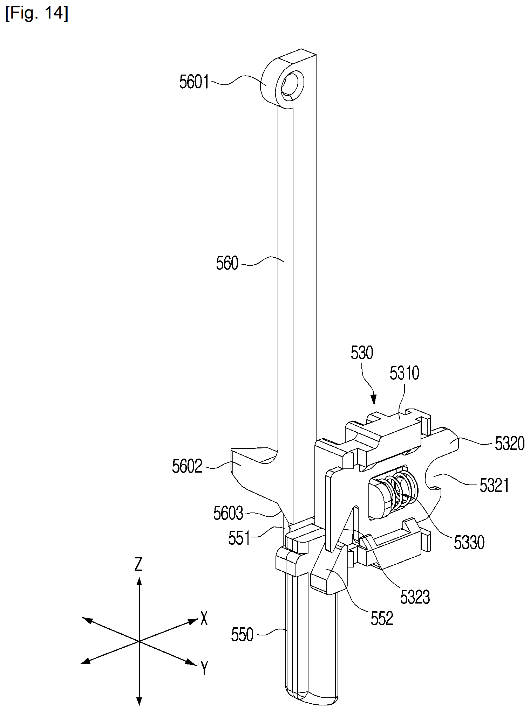

FIG. 14 is a view provided to explain a configuration for attaching or detaching the housing according to an embodiment. For the convenience of explanation, only the disassembly button 550, a securing bar 560, and the sliding member 530 which are installed in the internal housing 50 are illustrated.

Referring to FIGS. 10 and 14, the internal housing 50 may include the securing bar 560 which is disposed adjacent to a moving path through which the sliding member 530 moves in the vertical direction, and the disassembly button 550 which is disposed under the securing bar 560. In an embodiment, the securing bar 560 may have an elongated bar shape and have an upper end 5601 coupled to the internal housing 50 and have an inclined surface 5603 formed at a lower end thereof as an opened end. In addition, a securing piece 5602 may be formed at a position adjacent to the lower end of the securing bar 560, and may protrude by a length to penetrate through a penetrating hole 53 of the internal housing 50. The penetrating hole 53 of the internal housing 50 may be formed at a position aligned with a recess (17 of FIG. 6A) of the case 10 of the hair transplanter main body A, and accordingly, the securing piece 5602 may be inserted into the recess 17 of the hair transplanter main body A, such that the electrification module C is secured to the hair transplanter main body A.

As shown in FIG. 14, an inclined surface 5603 may be formed on a lower end of the securing bar 560, and an inclined surface 551 may be formed on an upper portion of the disassembly button 550 to face the inclined surface 5603. Accordingly, when a user presses up the disassembly button 550, the securing piece 5602 of the securing bar 560 moves along the Y axis in a direction away from the recess 17 of the hair transplanter main body A, such that the electrification module C and the hair transplanter main body A are decoupled from each other.

Since the locking projection 1451 of the connection member 1450 is inserted into the locking recess 5321 of the locking plate 5320 of the sliding member 530 when the electrification module C is secured to the hair transplanter main body A, the locking recess 5321 of the locking plate 5320 and the locking protrusion 1451 should be decoupled from each other in order to disassemble the electrification module C from the hair transplanter main body A. In an embodiment, to achieve this, an inclined surface 552 may be formed on an upper portion of the disassembly button 550, and an inclined portion 5323 may be formed on an area of the locking plate 5320 with which the inclined surface 552 interferes. Accordingly, when the disassembly button 550 moves up, the locking plate 5320 moves along the X axis in a direction away from the locking projection 1451.

Accordingly, according to the above-described configuration, when a user pushes up the disassembly button 550, the securing piece 5602 of the securing bar 560 may be released from the recess 17 of the hair transplanter main body A, and simultaneously, the locking plate 5320 may slide and move away from the locking projection 1451, and in this case, the user may disassemble the electrification module C from the hair transplanter main body A by lifting the electrification module C.

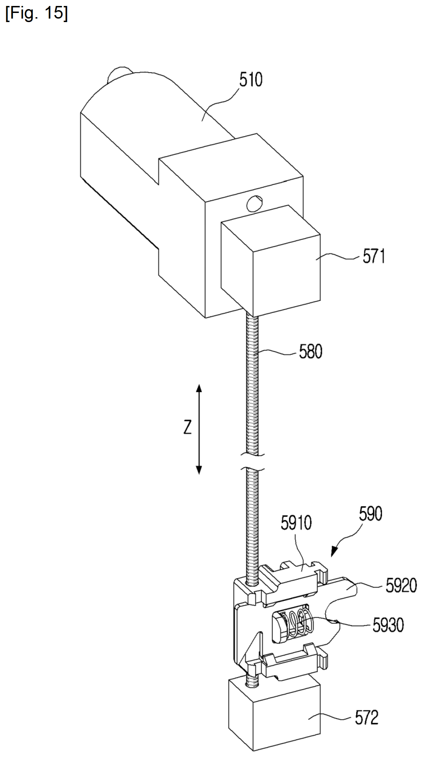

FIG. 15 illustrates an alternative embodiment for driving the sliding member 530, and illustrates only components needed to drive the sliding member 530 similarly to FIG. 11.

Referring to FIG. 15, in this alternative embodiment, the internal housing 50 may include a gear box 571 which is connected to the driving shaft of the driving unit 510, a screw shaft 580 which extends downwards from the gear box 571, and a support 572 to support a lower end of the screw shaft 580, and a sliding member 590 which moves in the vertical direction by means of the screw shaft 580. That is, in this alternative embodiment, the sliding member 590 is moved by using the screw shaft rather than a pully and a wire.

The sliding member 590 may include a main body 5910, a locking plate 5920, and a spring 5930. The locking plate 5920 and the spring 5930 have the same or similar configuration as or to the sliding member of FIGS. 10 to 14, and thus a detailed description thereof is omitted. The main body 5910 may be configured to allow the screw shaft 580 to penetrate therethrough, and may have the same configuration as the main body 5310 of FIGS. 10 to 14 except that the main body 5910 moves up and down by rotation of the screw shaft 580.

Accordingly, the screw shaft 580 may be rotated by driving by the driving unit 510 according to the above-described configuration, and accordingly, the sliding member 590 may move in the vertical direction, thereby raising and lowering the elevation gear holder 1400.

Various modifications and changes can be made by those skilled in the art based on the above descriptions of the disclosure. Therefore, the scope of the present disclosure is defined not by the detailed description of the embodiments but by the appended claims and equivalents thereto.

## Claims

1. A multi-channel hair transplanter provided with a plurality of needle channels, the multi-channel hair transplanter comprising:
a needle channel bundle B comprising a bundle core member 1100 and a plurality of needle channels which are slidably coupled to a radial outside of the bundle core member;
a hair transplanter main body A configured to accommodate the needle channel bundle B; and
an electrification module C attachably and detachably coupled to the hair transplanter main body A.

2. The multi-channel hair transplanter of claim 1, wherein the hair transplanter main body A comprises:
a cylindrical case 10 configured to accommodate the needle channel bundle B; and
an elevation gear holder 1400 interposed between the needle channel bundle B and the case 10 and movable in a vertical direction relative to the case 10.

3. The multi-channel hair transplanter of claim 2, wherein the electrification module C comprises:
a driving unit;
a first rotation member connected to a rotation shaft of the driving unit;
a second rotation member installed below the first rotation member, spaced apart from the first rotation member;
a power transmission member wound around the first and second rotation members to form a closed loop, and configured to transmit a rotational power of the first rotation member to the second rotation member; and
a sliding member coupled to the power transmission member to be movable in the vertical direction by driving by the driving unit.

4. The multi-channel hair transplanter of claim 3, wherein the first rotation member and the second rotation member are pullies, respectively, and the power transmission member is a wire.

5. The multi-channel hair transplanter of claim 3, wherein the first rotation member and the second rotation member are sprockets, respectively, and the power transmission member is a chain.

6. The multi-channel hair transplanter of claim 2, wherein the electrification module C comprises:
a driving unit;
a screw shaft disposed in the vertical direction and rotated by driving by the driving unit; and
a sliding member slidably coupled to the screw shaft and movable in the vertical direction by driving by the driving unit.
